Europäisches Patentamt

European Patent Office

Office européen des brevets

(11) Publication number: **0 319 686**

**A2**

## (12) EUROPEAN PATENT APPLICATION

(21) Application number: **88117479.1**

(22) Date of filing: **20.10.88**

(51) Int. Cl.⁴: **G01N 33/574 , G01N 33/545**

(30) Priority: **21.10.87 JP 265883/87**

(43) Date of publication of application:
**14.06.89 Bulletin 89/24**

(84) Designated Contracting States:
**AT BE CH DE ES FR GB GR IT LI NL SE**

(71) Applicant: **MOCHIDA PHARMACEUTICAL CO., LTD.**
**7, Yotsuya 1-chome**
**Shinjuku-ku Tokyo 160(JP)**

(72) Inventor: **Mochida, Ei**
**5-4, Komagome 2-chome**
**Toshima-ku Tokyo(JP)**
Inventor: **Inaji, Hideo**
**1-1-204, Yamamoto-Minami 3-chome**
**Takarazuka-shi Hyogo(JP)**
Inventor: **Mori, Takesada**
**19-512, Ryodo-cho 2-chome**
**Nishinomiya-shi Hyogo(JP)**

(74) Representative: **Henkel, Feiler, Hänzel & Partner**
**Möhlstrasse 37**
**D-8000 München 80(DE)**

(54) **Method for assaying a target substance in a minute amount of nipple discharge and device for such an assay.**

(57) Concentration of the target substance in the nipple discharge is assayed by means of an antigen-antibody reaction using an antibody and/or a fragment thereof against said target substance, and said antigen-antibody reaction is effected on a sheet-form solid phase.

The assay of the concentration of the target substance in the nipple discharge is effected by use of a device comprising a sheet-form solid phase having at least one reaction area defined thereon for an antigen-antibody reaction between an antibody and the target substance in the nipple discharge.

EP 0 319 686 A2

## Method for Assaying a Target Substance in a Minute amount of Nipple Discharge and Device for Such an Assay

### BACKGROUND OF THE INVENTION

Technical Field

This invention relates to a method for assaying a target substance, particularly a tumor-associated antigen in a very minute amount of nipple discharge, and a device for carrying out such an assay.

Background Art

Quite numerous tumor markers, namely, tumor-associated antigens are currently reported, and their concentration in blood are also assayed. Among these markers, the most widely employed tumor-associated antigen is CEA (carcinoembryonic antigen) which was reported by Gold et al. (J. Ext. Med., 121, 439, 1965). Assay of CEA concentration in blood is found to be useful for monitoring patients after surgery and determining effects of various treatments. The concentration of such a tumor-associated antigen is assayed by quantitative reactions including radioimmunoassay (RIA) and enzyme immunoassay (EIA). It is, however, very difficult to detect a cancer at an early stage by measuring the concentration of the tumor-associated antigen in blood including CEA.

Although breast cancer is one of the most potent cancers which are found to be gradually increasing, sensitibity obtained by the assays of the tumor-associated antigens in blood has been quite low regardless of the type of the tumor-associated antigen employed, and the detection of primary cancer at an early stage has been extremely difficult. Consequently, there have been many cases wherein the cancer had proceeded too far and metastasis had occurred to such a site as lymph node and the like, inevitably requiring treatments such as mastectomy.

Inaji et al. (Igaku-no-Ayumi, 134, 575, 1985) reports that breast cancer can be detected at an early stage by measuring CEA which is a tumor-associated antigen found in nipple discharge. In this process, the assay of CEA in nipple discharge is carried out by enzyme immunoassy (Elmotech-CEA, Mochida Pharmaceutical Co., Ltd.). This CEA assay, however, requires various equipments including a reaction equipment and a spectrophotometer for determining the results of the reaction, and therefore, such a CEA assay is far from a convenient process which can be readily carried out at every facility. Further, since a calibration curve must be depicted at every examination regardless of the number of the specimens, this process has been inefficient for such a symptom as abnormal secretion from nipple wherein the specimen is less frequently generated. Moreover, an amount of at least 50 microliters of specimen is required even for Elmotech-CEA, which is an EIA kit employed for measuring CEA presently requiring the relatively small amount of specimen. The amount of nipple discharge secreted is usually as small as no more than 10 microliters. The nature of the nipple discharge, particularly the viscosity, is also widely varied among specimens compared to serum specimens, and the collection of the specimen is difficult in some cases. The amount of the specimen collected has often been too small to carry out the assay, and a development of an assay which can be carried out for a minute amount of specimen is strongly desired.

In consideration of such a background, there is a marked social demand for a development of a highly specific and potent diagnosis of the breast cancer. Particularly desired is a development of an assay of a tumor-associated antigen which can be utilized in a massscreening for breast cancer at group examination or dispensary of mammary gland. The assay of the tumor-associated antigen, particularly CEA, in nipple discharge has a significant importance in such field, and a convenient procedure for accurately measuring the tumor-associated antigen in nipple discharge which can be carried out by anyone would certainly have a clinical significance.

After making an intensive study to overcome the above-described problems in the diagnosis of breast cancer and to develop a method for measuring a target substance in nipple discharge, especially a tumor-associated antigen in nipple discharge, which can meet the social demands to carry out the assay using a minute amount of specimen at group examination or mass screening by a simple operation, the inventors of the present invention have found that the above-mentioned problems may be overcome by utilizing a sheet-form material as a carrier for the insolubilized antibody used in conventional EIA. The present invention is

EP 0 319 686 A2

achieved on such a finding.

## SUMMARY OF THE INVENTION

An object of the present invention is to provide a method for conveniently detecting or assaying a target substance in a biological discharge, especially a tumor-associated antigen in a minute amount of nipple discharge by an immunoassay using an antigen-antibody reaction as well as a device required to carry out such a method. A further object of the present invention is to enable the detection of breast cancer at an early stage and to meet the above-mentioned social demand.

According to a first aspect of the present invention, there is provided a method for assaying a target substance in a minute amount of nipple discharge wherein concentration of the target substance in the nipple discharge is assayed by means of an antigen-antibody reaction using an antibody or a fragment thereof against said target substance, and said antigen-antibody reaction is effected on a sheet-form solid phase.

According to a second aspect of the present invention, there is provided a device for assaying a target substance in a minute amount of nipple discharge comprising a sheet-form solid phase having at least one reaction area defined thereon for an antigen-antibody reaction between an antibody and the target substance in the nipple discharge.

The sheet-form solid phase may preferably be a substantially liquid impermeable plastic sheet selected from the group consisting of polyester, polypropylene, and a polyethylene.

## BRIEF DESCRIPTION OF THE DRAWINGS

FIG. 1 is a diagram illustrating the concentration of CEA in nipple discharge in various diseases examined in Example 2.

FIG. 2a is a plan view, and FIG. 2b is a cross-sectional view of one embodiment of the device according to the present invention.

FIG. 3a is a plan view, and FIG. 3b is a cross-sectional view of another embodiment of the device according to the present invention.

FIG. 4a is a plan view, and FIG. 4b is a cross-sectional view of a further embodiment of the device according to the present invention.

FIG. 5a is a plan view, and FIG. 5b is a cross-sectional view of a still further embodiment of the device according to the present invention.

## DETAILED DESCRIPTION OF THE INVENTION

The method for assaying a target substance in a minute amount of nipple discharge is characterized in that the assay is carried out on a sheet-form solid phase. The employment of the sheet-form solid phase has enabled a minute amount of the nipple discharge to be assayed for the target substance. The term "minute amount" used herein designates an amount in the order of from 0.2 to 50 microliters. The nipple discharge collected from patients may be used either with or without dilution by a factor of about 2 to 100.

The method according to the present invention is preferably based on a sandwich method comprising the steps of applying the nipple discharge onto a substantially liquid impermeable sheet-form solid phase onto which an antibody or a fragment thereof (the antibody and the fragment thereof are hereinafter generically referred to as antibody) against the target substance in the nipple discharge to be detected or assayed is applied to react the specimen with the insolubilized antibody; applying a labelled substance comprising said antibody against said target substance having a labelling agent which can be identified by appropriate means bound thereto onto said solid phase to allow for a reaction; thereafter removing portion of the nipple discharge and the labelled substance which failed to bind with the antibody insolubilized onto said solid phase by rinsing the solid sheet; and measuring the concentration of the target substance from the amount of the labelled substance bound to said sheet-form solid phase. The present method, however, is not limited to such a sandwich method.

3

An exemplary method which is not based on the sandwich method employs a sheet-form solid phase without any antibody insolubilized thereon. In such a method, the collected specimen is applied onto a predetermined area on the solid phase having no antibody insolubilized thereon; a labelled substance comprising the antibody having an identifiable labelling agent bound thereto is applied onto the solid phase to allow for reaction; the solid phase is then rinsed to remove the specimen components and the labelled substance which failed to bind with the solid phase; and the amount of the target substance is assayed from the labelled substance bound onto said predetermined area of the solid phase.

In the sandwich method, the antibody is less likely to be influenced by the components in the nipple discharge other than said target substance since the target substance of the nipple discharge is immunochemically bound onto the area on the solid phase wherein the the antibody is insolubilized. Further, the sandwich method is more sensitive than the method utilizing the solid phase with no antibody insolubilized thereto. The adjustment of the sensitivity and the measurable concentration range may also be more readily carried out in the sandwich method. The sandwich method wherein the antibody is insolubilized onto the solid phase is thus desirable.

Three variations of the sandwich method can be carried out in the present invention corresponding to the reaction order of three factors, that is, the antibody insolubilized onto the sheet-form solid phase, the specimen, and the labelled substance.

The first method comprises the steps of applying the nipple discharge specimen onto the sheet-form solid phase having the antibody against the target substance in the nipple discharge to be detected or assayed bound thereto to allow for a reaction between the specimen and the insolubilized antibody; applying the labelled substance comprising the antibody against said target substance having the labelling agent identifiable by appropriate means bound thereto onto said solid phase to allow for a reaction; rinsing said solid phase to remove the nipple discharge components and the labelled substance which failed to bind with the antibody insolubilized onto the solid phase; and measuring the concentration of the target substance from the amount of the labelled substance bound to the sheet-form solid phase.

The second method comprises the steps of preliminary applying the labelled substance comprising the antibody having the identifiable agent bound thereto onto the sheet-form solid phase having the antibody insolubilized thereon; and applying the nipple discharge specimen on the predetermined area of the thus pretreated solid phase to simultaneously effect the reaction between the specimen components and the insolubilized antibody and the reaction between the specimen components and the labelled substance. In this method, an optional step of lyophilization carried out after the application of the labelled substance onto the solid phase having the antibody insolubilized thereon may increase stability of the reagents including the labelled substance.

The third method which may be carried out in the present invention comprises the steps of preliminary mixing the collected nipple discharge specimen and the labelled substance comprising the antibody having the identifiable labelling agent bound thereto, and applying said mixture on the predetermined reaction area of the solid phase having the antibody insolubilized thereon to allow for a reaction.

Any substance which is measurable by the antigen-antibody reaction may be used as the target substance in the assay of the present invention. Typical target substances which may be used include tumor-associated antigen whose concentration in blood has conventionally been assayed, such as CEA (carcinoembryonic antigen), AFP (alpha-fetoprotein), TPA (tissue polypeptide antigen), tummor-associated antigens (for example, CA19-9, CA125, CA15-3, Milk Fat Globule Antigens, TAG72 and F36/22 Antigen) such as those identified by other monoclonal antibodies, as well as hormone receptors, such as estrogen receptor and progesterone receptor, and pathogenic microorganism antigens. The antibody against such a target substance may either be produced in a conventional manner or purchased from a suitable source.

The antibodies used in the method according to the present invention are not limited to purified antibodies but include crude antibodies such as an antiserum and a salted out antibody containing an antibody having binding activity with said target substance. The antibody fragments which may be employed include the antibody moieties having binding activity with the target antigen such as Fab and F-(ab)$_2$. The antibody may either be polyclonal or monoclonal, and an appropriate antibody may be selected depending on the nature of the target substance to be assayed.

The antibody may either be physically or chemically bound to the solid phase. For example, the solid phase may be immersed and incubated in a solution containing the antibody at 4 to 56$^\circ$C for 0.5 to 24 hours, and more preferably at 37 to 50$^\circ$C for 1 to 2 hours to immobilize the antibody onto the solid phase. The concentration of the antibody employed may be in the range of from 1 microgram to 100 mg/ml and preferably from 1 to 100 micrograms/ml when the antibody is a purified antibody or a purified antibody fragment. The solid phase which has been immersed in the antibody solution may either be air-or freeze-dried. Insolubilization of the antibody onto the solid phase is not limited to the above-described manner, and

4

all processes are included in the present invention wherein the antibody is immobilized onto the solid phase to substantially prevent the antibody from being separated from the solid phase in course of the conventional assay including the coating and rinsing operation.

The sheet-form solid phase having the antibody immobilized thereon may preferably be coated with a substance which does not participate in the antigen-antibody reaction between the antibody and the target substance in the nipple discharge in order to suppress non-specific reactions and more easily determine the result of the reaction. Such substances which do not participate in the antigen-antibody reaction may typically be a thermally denatured cytochrome c solution, BSA (bovine serum albumin), HSA (human serum albumin), animal serum, skim milk, and the like.

Amount of the antibody insolubilized onto the reaction area is determined in accordance with the concentration of the target substance to be assayed in the nipple discharge. For example, in a system wherein all the specimens containing the target substance above the predetermined concentration (cut off concentration) are to be determined positive, a convenient and accurate determination may be carried out by insolubilizing onto the solid phase such an amount of the antibody that all the antigen-binding sites of the antibody insolubilized within the reaction area would be substantially saturated or occupied by the target substance at the cut off concentration. In another example wherein the target substance contained in the specimen is to be quantitatively or semi-quantitatively assayed within a predetermined concentration range, a convenient and strict determination may be carried out by insolubilizing onto said reaction area such an amount of the antibody that all the antigen-binding sites of the antibody insolubilized onto said reaction area would be substantially saturated or occupied by the target substance contained in the specimen at the concentration at the upper limit of said range.

The labelled substance which may be employed in the method according to the present invention comprises the antibody or the fragment thereof against the target substance in the nipple discharge which has an identifiable labelling agent bound thereto.

The identifiable labelling agents which may be bound to the antibody include pigment, radioisotope, enzyme and fluorescent material, among which the enzyme being preferred in view of stability, detection sensitivity, safety, and ease of treatment. The enzymes which may be employed include horseradish peroxidase, alkaline phosphatase, and the like, which are employed in conventional enzyme immunoassays. The result of the reaction may be determined by assaying the amount or the activity of the labelled substance bound to the target substance on the solid phase after the immunoreaction. When an enzyme is used for the identifiable labelling agent, the assay of the labelled substance may be carried out by measuring the amount of the resulting reaction product of the labelling enzyme. The amount of the reaction product may be assayed quantitatively by physical means using such measuring instruments as colorimeter, fluorophotometer, luminometer, and the like. When the reaction product is a chromophoric substance, the color developed may be either semi-quantitatively differentiated to several levels with the naked eye depending on its color strength, or qualitatively differentiated into positive and negative groups, the latter being more convenient. The chromophoric substance whose color may be developed by the enzyme reaction may be any substance which are employed in conventional enzyme immunoassays. Illustrative chromophoric substances which may be used with peroxidase include 2,2′-aminodi(3-ethylbenzthiazoline)-6′-sulfonic acid (ABTS), o-phenylenediamine (OPD), tetramethylbenzidine (TMB), and 5-aminosalicylic acid. In the case of alkaline phosphatase, the enzyme may be reacted with such a substrate as p-nitrophenol phosphoric acid or phenyl phosphoric acid, and a phenol resulting from such an enzyme reaction may oxidatively conjugated with 4-aminoantipyrine to allow for color development. Useful chromophoric substances are provided with properties that they deposit on the sheet-form solid phase and their color tint do not significantly change with the lapse of time, since the resulting sheet can be stored after the determination of the result. Various chromophoric substances having such properties are known including diaminobenzidine which may be used with peroxidase, and 5-bromo-4-chloro-3-indoryl phosphonate (BCIP) which may be used with alkaline phosphatase.

In the method according to the present invention, the nipple discharge is usually collected with a suction pipette or a capillary, and the collected specimen is applied onto a predetermined reaction area of the solid phase. By using such procedure, the site of the disease can be estimated on the basis of the position of the mammary grand from which the particular specimen has been collected. In the case of collecting an extremely minute amount of nipple discharge, the specimen may be collected directly from the nipple onto the reaction area of the solid phase.

A substantially liquid-impermeable sheet-form solid phase is used as a carrier on which the antibody against the target substance is insolubilized, and the antigen-antibody reaction as well as the quantitative assay of the labelled substance are effected. The materials which may be used for such a solid phase include plastic resins such as polyester, polypropylene, vinyl chloride, polyethylene, polymethylpentene, etc., synthetic films such as Nylon film, nitrocellulose film, etc., and a paper coated with above-mentioned materials. Among those, plastic resins such as polyester, polypropylene and polyethylene, as well as Nylon film are particularly preferred since they rarely suffer from non-specific adsorption of components in the nipple discharge other than the target substance, and they may be readily handled during the assaying process including the rinsing. The liquid-impermeable sheet-form solid phase allows for an extremely minute amount of the specimen to be applied onto a relatively large area of the solid phase enabling the specimen to become in contact with a larger number of antibody molecules. At the same time, such a solid phase enables the specimen to be coated to a thinner layer allowing for a higher reaction efficiency. Effects of viscosity of the specimen on the assay results may also be minimized since the specimen applied onto the sheet-form solid phase exhibits little non-specific adsorption and may readily be removed by rinsing before or after the drying and solidifying of the reaction product on the solid phase regardless of the viscosity of the specimen. Consequently, effects on the result of the solidification of the reaction products or the nature of the specimen are minimized. By taking advantage of such properties of the liquid-impermeable solid phase, the solid phase having the specimen applied on the reaction area thereof may be stored until a satisfactory number of the specimens have been collected for further process. Such storage of the solid phase having the specimen applied thereon realizes an increased degree of freedom in such a process as mass-screening wherein a large number of specimens are irregularly collected. The above-described effects may be enhanced by using a substantially liquid-impermeable plastic resin for the material of the sheet-form solid phase.

The specimen which may be used for the assay of the present invention is not essentially limited to the nipple discharge. Other biological liquids such as serum, plasma, urine, saliva, tear, perspiration, smear, and the like may also be employed for the assay according to the present invention, although the present method is most effective for a minute amount of specimen such as nipple discharge as apparent from the above-described effects.

The antibody employed in the method of the present invention is more specifically described by referring to a case wherein the target substance in the nipple discharge is CEA.

An anti-CEA antibody should be highly specific to CEA since there is an antigen analogous to CEA such as NCA (non-specific cross-reacting antigen) which has an antigenic determinant in common with CEA. The inventors of the present invention have succeeded in obtaining a highly specific anti-CEA antibody by preparing a numerous samples of monoclonal anti-CEA antibodies in a trial-and-error manner, selecting the antibody samples with respect to reactivity with CEA, further selecting the thus selected antibody samples with respect to the specificity to CEA, and certifying the repeatability of such properties.

The procedure for selecting such an antibody is described below.

(a) Preparation of the Anti-CEA Antibody

A mixture of 50 micrograms of purified CEA and complete Freund adjuvant was subcutaneously administered to BALB/c mouse four times at an interval of one week, and spleen cells were collected four days after the last administration. The thus collected $1 \times 10^7$ spleen cells were fused with $1 \times 10^8$ mouse myeloma cells (P3U1) by a conventional method. HAT medium (RPMI-1640 medium containing hypoxanthine, aminopterin, thymidine, and 10% fetal bovine serum) was added to these cells until the total volume was 20 ml, and this culture medium was dispensed into 0.2 ml aliquot per well on a 96-well microplate and cultivated for two weeks. Supernatants of the thus propagated cultures in the well were measured for their antibody activity. The cells from the active wells were added to 40 ml of RPMI-1640 medium having 10% fetal bovine serum added thereto containing thymic cells of BALB/c mouse. This cell suspension culture was dispensed into two 96-well microplates, cultivated for one week to obtain 51 cell lines of anti-CEA antibody-producing hybridoma. These cell lines were cultivated on a large scale and purified by means of chromatography using DEAE-cellulose column to obtain immunoglobulin fractions.

(b) Selection of anti-CEA antibody

0.2 ml portions of the 51 anti-CEA antibody obtained in (a) were respectively dispensed into a 96-well microtiter plate at a concentration of 10 micrograms/ml, and incubated overnight at 4°C for allowing an adsorption reaction to obtain insolubilized antibodies.

In the meanwhile, horseradish peroxidase was bound to the 51 anti-CEA antibodies obtained to produce labelled antibodies by a method of Kawaoi (J. Histochem. Cytochem., 22: 1084, 1974). The 51 insolubilized antibodies and the 51 enzyme-labelled antibodies were mutually combined, and reacted with purified CEA by a sandwich-type EIA to examine 2601 combinations. The results are shown in Table 1.

Table 1

**+** : strong reaction
**+** : weak reaction
**−** : no reaction

Among these 51 antibodies, 24 antibodies which exhibited a strong reaction when employed as either the insolubilized antibody or the labelled antibody, that is, antibody Nos. 1, 2, 3, 4, 5, 6, 8, 9, 10, 11, 12, 13, 14, 15, 16, 17, 18, 19, 20, 30, 37, 38, 39, and 44 were selected. These 24 antibodies were examined by observing their reactions with tissues from colorectal cancer, normal large intestine, stomach cancer, normal stomach, breast cancer, and glanulocyte tissue by using immunohistological staining, and 3 antibodies exhibiting high specificty for cancer, that is, antibody Nos. 1, 6, and 10 were selected. By reacting these 3 antibodies with CEA which has been treated with periodic acid, antibody No. 1 was identified to be an antibody reacting with a sugar chain portion of CEA molecule, and antibody Nos. 6 and 10 were identified to be antibodies reacting with a peptide portion of CEA molecule.

When CEA in the nipple discharge was measured by the method according to the present invention using the anti-CEA antibody obtained by the above-described procedure, many patients with an early stage cancer exhibiting no CEA in blood by conventional CEA detecting method often exhibited an extraordinary high CEA value in the nipple discharge. This indicates that the method for assaying the tumor-associated antigen in the nipple discharge according to the present invention can serve a potent auxiliary diagnostic means. Further, the method according to the present invention has a clinical significance since no measuring apparatus is required like conventional method for assaying tumor-associated antigen in blood, and a simple collection of the nipple discharge and CEA assay are realized allowing the assay to be carried out at all facilities.

Detection of the breast cancer at an early stage has been very difficult, and consequently, the cancer has frequently proceeded to far and gave rise to metastases to lymph nodes before it was detected inevitably requiring a large-scale operation such as mastectomy. In the case of the breast cancer which is detected at its early stage by the assay of CEA in the nipple discharge according to the present invention, the breast cancer may be treated by a relatively simple operation such as microdochectomy allowing a relatively favorable process after the operation.

For example, the sensitivity obtained in the conventional assay of CEA in blood has been 25 to 70% in relapsed breast cancer and advanced stage breast cancer at stage IV whereas the sensitivity has been less than about 10% in primary breast cancer at stage I and stage II (H. Sonooi et al., Clinical Adult Diseases, vol. 16, No. 6, 1007-1014, 1986; T. ITO, Cancer and Chemotherapy, vol. 14, No. 10, 2917-2923, 1987). The sensitivity is even lower in breast cancer exhibiting no palpable tumor. In the assay of CEA in the nipple discharge according to the present invention, the sensitivity was 83% in the nonpalpable breast cancer, the specificity was 78%, and the accuracy was 79% when a cut off value of 200 ng/ml was used. Since the method according to the present invention can be carried out on one solid phase from the collection of the specimen to the assay, it may be conveniently carried out, assayed at an appropriate time after the collection by storing the collected specimen, and has a high sensitivity. When a patient is found to exhibit an abnormal nipple discharge in a dispensary of the hospital or at a group examination, the assay of the target substance including a tumor-associated antigen in the nipple discharge may conveniently be carried out to allow a doctor to quickly grasp the cause of the symptom and give an appropriate treatment.

The device for assaying the target substance in the nipple discharge according to the present invention is described in detail with reference to the preferred embodiments illustrated in the drawings.

The sheet-form solid phase may have any practically convenient configuration and size so long as at least one reaction area having a predetermined size can be provided thereon. Four non-limiting preferable examples are set forth below.

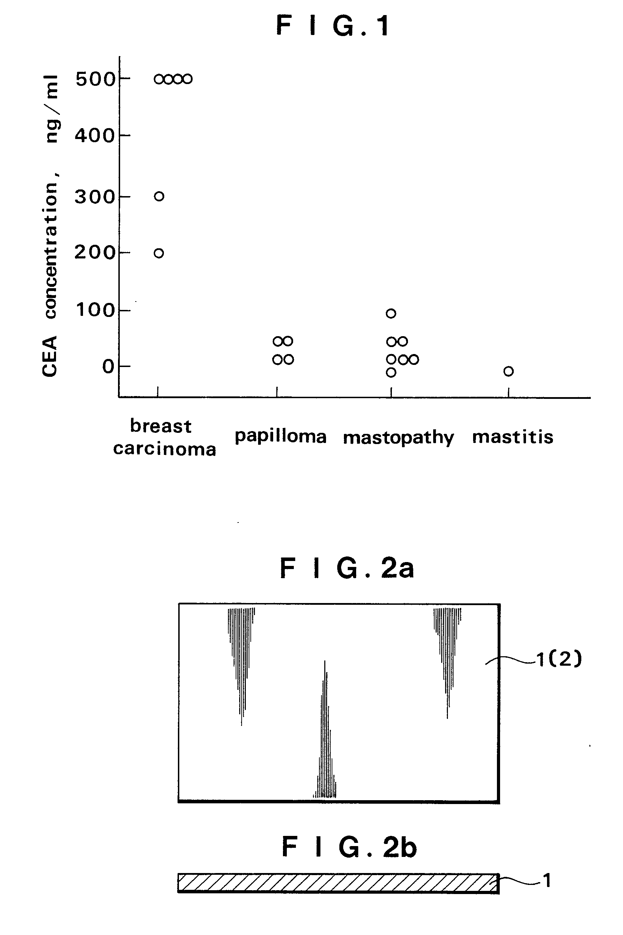

FIG. 2a is a plan view, and FIG. 2b is a cross-sectional view of a sheet 1 having one reaction area 2 thereon. The sheet 1 may preferably have a thickness of 0.1 to 2 mm, and any convenient size may be used to meet the handling requirements.

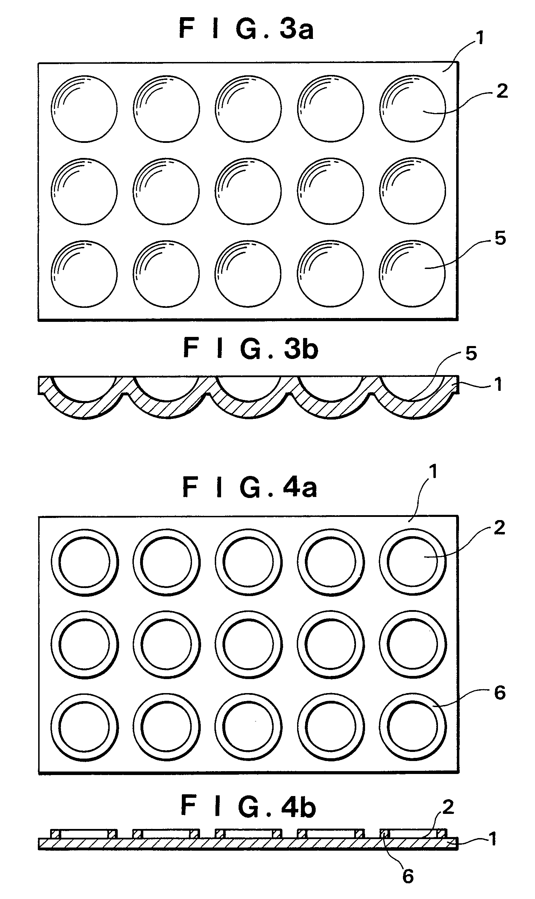

FIG. 3a is a plan view, and FIG. 3b is a cross-sectional view of the sheet 1 having a plurality of reaction areas 2. The reaction area 2 in this embodiment is a recess 5 provided in the sheet 1.

FIG. 4a is a plan view, and FIG. 4b is a cross-sectional view of the sheet 1 having a plurality of reaction areas 2. The reaction area 2 in this embodiment is surrounded by a ridge or frame 6.

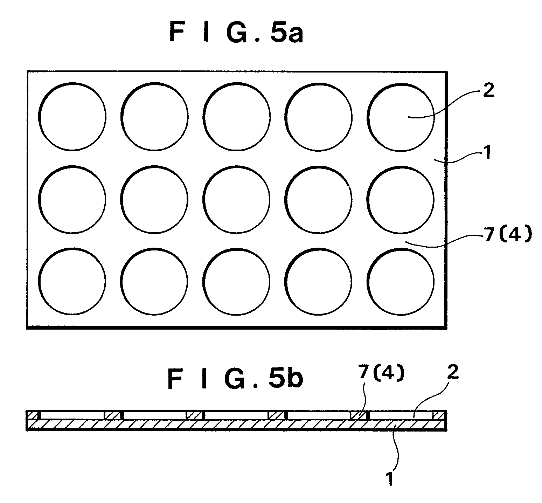

FIG. 5a is a plan view, and FIG. 5b is a cross-sectional view of the sheet 1 having a plurality of reaction areas 2. The reaction areas 2 in this embodiment are defined by a frame 7 covering the entire top surface of the sheet except for the reaction areas 2.

The size and the shape of the reaction area may be selected in accordance with the amount of the nipple discharge applicable thereon. For example, 5 microliters of the specimen may generally applied to an area of 3.0 to 0.01 cm$^2$. This area corresponds to a circle having a diameter of about 20 to 1 mm, although a diameter of 15 to 2 mm is preferable for a convenient operation and an easy determination. Of cause, these values may vary in accordance with the amount of the specimen used for the assay. The amount of the specimen required may also vary in accordance with the sensitivity of the assay and the range of the concentration to be assayed, and the size of the reaction area is accordingly varied. The non-limiting shape of the reaction area may preferably be a circle, ellipse, square, rectangle, and other quadrilateral for the purpose of a convenient operation including the application and rinsing as well as an easy determination.

The reaction area may be defined by merely insolubilizing the antibody onto a predetermined limited area of the solid phase, although it is more preferable that the predetermined reaction area of the solid phase is surrounded by a frame or a ridge provided on the solid phase or a recess provided in the sheet-form solid phase. The provision of such an area may readily be carried out when the solid phase is prepared from a substantially liquid-impermeable plastic resin.

8

The provision of the ridge or the frame surrounding the predetermined reaction area on the sheet-form solid phase may be carried out by press molding the ridge or by printing the frame with a water-repellent paint.

The reaction area may also be defined by covering the top surface of the solid phase except for the reaction area by means of a frame comprising an appropriate material such as aluminum foil 4 as shown in FIGS. 5a and 5b. More illustratively, such provision of the reaction area may be carried out by preparing an aluminum foil having an appropriate thickness in the range of 0.05 to 1.0 mm having openings whose size and shape are consistent with those of the reaction area; and heat bonding the aluminum foil onto the solid phase by using a hot-melt adhesive.

The reaction area may also be provided as a recess by such means as press molding as shown in FIGS. 3a and 3b.

It is to be noted that the reaction area of the predetermined size may also be provided by various means other than those described above.

The present invention is further illustrated by the following non-limiting examples.

## EXAMPLES

Example 1: Comparison among various materials of the sheet-form solid phase carriers to which the antibody is immobilized

(a) Purification of the antibodies

The 2 cell lines of hybridomas respectively producing a monoclonal antibody whose high specificity for cancer has been proven (hereinafter referred to as antibody Nos. 1 and 10) obtained in the above-described cell fusion procedure were cultivated. Immunoglobulin fractions were separated from 10 liters of the culture supernatant of each of the hybridomas by ammonium sulfate precipitation method. These fractions were respectively purified by means of affinity chromatography on a Protein A Sepharose column to obtain antibody Nos. 1 and 10. Antibody No. 1 was dialyzed against 0.01 M carbonate buffer solution, pH 9.5 (hereinafter referred to as SCB), and antibody No. 10 was dialyzed against 76 mM phosphate buffer saline, pH 6.4 hereinafter referred to as PBS) to obtain 279 mg of antibody No. 1 and 134 mg of antibody No. 10.

(b) Preparation of the enzyme-labelled antibody

A 5 mg portion of horseradish peroxidase (grade 1-C, TOYOBO K.K., hereinafter referred to HRPO) was dissolved in 1 ml of 0.3 M aqueous solution of sodium hydrogencarbonate, and 0.1 ml of 1% ethanol solution of dinitrofluorobenzene was added to the solution and allowed to react at room temperature for 1 hour. 1 ml of 60 mM sodium periodate solution was then added to the solution and allowed to react for 30 minutes, and 1 ml of 0.16 M ethylene glycol solution was then added and allowed to react for 1 hour, and the thus obtained solution was dialyzed against SCB. To this solution, 5 mg of antibody No. 1 purified in the above-described procedure (a) was added and allowed to react at room temperature for 3 hours. 5 mg of sodium boronhydride was added to the solution and allowed to react overnight at 4°C, and the thus obtained solution was dialyzed against PBS to obtain an HRPO-labelled anti-CEA antibody (hereinafter referred to as HRPO-labelled antibody No. 1).

(c) Insolubilization of the antibody

The solution of antibody No. 10 was adjusted to a concentration of 5 mg/ml with PBS. Various sheets (polyester, polypropylene, Nylon, nitrocellulose, filter paper, aluminum, iron, glass) were immersed in the solution, incubated at 45°C for 1 hour, and rinsed with distilled water. After thoroughly draining the water, the sheets were immersed in PBS to which 10% BSA has been added, and incubated at room temperature for 1 hour. The sheets were then thoroughly drained, air dried, and stored at 4°C.

9

(d) Comparison of the sheet-form solid phase carriers

Standard CEA solutions (5µL) at 0, 50, 100, 200, and 500 ng/ml were applied to five reaction areas of the antibody-insolubilized sheets prepared in the above-described procedure (c). After drying, 25 microliter portions of standard antibody No. 1 solution which has been diluted by a factor of a hundred with 1% BSA-added PBS were added dropwise to these sheet, and allowed to react at room temperature for 30 minutes. The sheets were washed with distilled water to remove the reaction solution, and further washed by violently agitating in a sealed container containing a cleaning solution (1 M saline to which 0.05% Tween 20 has been added). After thoroughly draining the sheet, 25 microliters of a substrate solution (0.5 mM TMB, 0.01% $H_2O_2$) were added dropwise onto the sheet and allowed to react for 10 minutes. The results are shown in Table 2 wherein the sample exhibiting color development is determined + and no color development is determined -, for each of the standard CEA solution at 0, 50, 100, 200, and 500 ng/ml. The best results were obtained when the polyester and polypropylene sheets were employed.

Table 2

| Comparison among Carriers | | | | | |
|---|---|---|---|---|---|
| Carrier | CEA, ng/ml | | | | |
| | 0 | 50 | 100 | 200 | 500 |
| Polyester | - | - | + | + | + |
| Polypropylene | - | - | + | + | + |
| Nylon | - | - | - | + | + |
| Nitrocellulose | - | - | - | + | + |
| Filter paper | - | - | - | - | + |
| Aluminum | - | - | - | - | - |
| Iron | + | + | + | + | + |
| Glass | - | - | - | - | - |

Example 2: Assay of CEA by using the polyester sheet having anti-CEA antibody immobilized thereto

(a) Preparation of the antibody-insolubilized sheet

An aluminum sheet having a plurality of circular openings with an inner diameter of 15 mm blanked at an interval of 15 mm was bonded to a polyester sheet (N500, manufactured by Somar K.K) by using a hot-melt adhesive. After fully washing and drying the thus prepared sheet, antibody No. 10 was insolubilized onto an area of the sheet which is not covered by the aluminum sheet by repeating the procedure of Example 1, (c) to prepare an antibody-insolubilized sheet.

(b-1) Assay of CEA

From patients suffering from abnormal nipple discharge, 18 specimens of the nipple discharge were collected and used for measuring CEA.

A 5 microliter portion of each of the specimens was respectively applied to cover the entire circular reaction areas of the antibody-insolubilized sheet prepared in (a) and dried. 25 microliters of HRPO-labelled antibody No. 1 which has been diluted by a factor of a hundred with 1% BSA-added PBS was added dropwise onto the dried sheet and allowed to react at room temperature for 30 minutes. The sheet was washed with distilled water to remove the reaction solution, and further washed by placing the sheet in a sealed container containing a cleaning solution (1 M saline to which 0.05% Tween 20 has been added) and violently agitating the cleaning solution. After thoroughly draining the sheet, 25 microliters of the substrate solution (0.5 mM TMB, 0.01% $H_2O_2$) was added onto the sheet and allowed to react for 10 minutes. The CEA concentration in each of the specimens was semiquantitatively measured by referring to the color developed using CEA standard solutions at concentrations of 0, 50, 100, 200, and 500 ng/ml as contrasts.

The results obtained by the method according to the present invention are shown in Table 3 together with the measurements of the same specimens obtained by using Elmotech-CEA. A good correlation was observed between the measurements of both methods. The measurements obtained by the method of the present invention are classified by the type of the disease, and diagramatically shown in FIG. 1. All specimens of breast cancer exhibited high values of over 200 ng/ml, and all specimens of benign diseases exhibited values of below 100 ng/ml.

Table 3

| Specimen | Present method (semi-quantitative) | Elmotech-CEA (quantitative) |
|---|---|---|
| A | 300 ng/ml | 285 ng/ml |
| B | 500 | 650 |
| C | 500 | 445 |
| D | 500 | 343 |
| E | 500 | 605 |
| F | 500 | 555 |
| G | 50 | 96 |
| H | 50 | 36 |
| I | 20 | 18 |
| J | 20 | 31 |
| K | 50 | 8 |
| L | 20 | 10 |
| M | 50 | 96 |
| N | 20 | 46 |
| O | 100 | 70 |
| P | 20 | 5 |
| Q | 0 | 11 |
| R | 0 | 0 |

(b-2) Effects of the storage period after the application of the specimen onto the solid phase

From patients suffering from abnormal nipple discharge, 3 specimens of the nipple discharge were collected and used for measuring CEA. The CEA concentration immediately after the collection were 0, 100, and 200 ng/ml, respectively.

5 microliters of each of the 3 specimens were applied to cover the circular reaction areas of the antibody-insolubilized sheet prepared in (a), dried and stored at 4° C. CEA concentration was assayed for each of the specimen by means of the procedure described in b-1). The results are shown in Table 4. A good correlation between the measurements were observed irrespective of the storage period.

Table 4

| CEA concentration after storage | | | |
|---|---|---|---|
| Storage period, day | CEA concentration, ng/ml | | |
| | 1 | 2 | 3 |
| 0 | 0 | 100 | 200 |
| 1 | 0 | 100 | 200 |
| 4 | 0 | 100 | 200 |
| 5 | 0 | 100 | 200 |
| 6 | 0 | 100 | 250 |
| 7 | 0 | 100 | 250 |
| 11 | 0 | 100 | 200 |
| 12 | 0 | 100 | 200 |
| 13 | 0 | 100 | 250 |
| 14 | 0 | 100 | 250 |

Example 3: Comparison among various methods for assaying CEA in nipple discharge

(a) Preparation of the antibody-insolubilized sheet

Circular frames having a width of 1 mm and an inner diameter of 6 mm were silk screen-printed at an interval of 10 mm on a polyester sheet (N500, manufactured by Somar K.K.) with white water-repellent paint. After thoroughly rinsing with water and drying the sheet, antibody No. 10 was insolubilized onto the sheet by repeating the procedure of Example 1, (c) to prepare the antibody-insolubilized sheet.

(b) Preparation of the enzyme-labelled antibody

Bovine intestine alkaline phosphatase (ALP, manufactured by Berlinger Manheim) at a concentration of 10 mg/ml was mixed with antibody No. 1 prepared by repeating the procedure of Example 1, (a) at a concentration of 5 mg/ml. Glutaraldehyde was added to the mixture to a final concentration of 0.2% and allowed to react at 4°C for 2 hours. The resulting solution was dialyzed against 50 mM Tris-HCl buffer solution, pH 8.0 containing 0.1 M sodium chloride, 1 mM magnesium chloride, and 0.1% sodium azide at 4°C to obtain an ALP-labelled anti-CEA antibody (hereinafter referred to as ALP-labelled antibody No. 1).

(c) Assay of CEA

From patients suffering from abnormal nipple discharge, 3 specimens of the nipple discharge were collected and used for measuring CEA.

(c-1) Procedure 1

A 5 microliter portion of each of three specimens was applied to cover the entire reaction area surrounded by the circular frame on the antibody-insolubilized sheet prepared in (a). After drying, 10 microliters of ALP-labelled antibody No. 1 was added dropwise and allowed to react at room temperature for 30 minutes. The sheet was washed with distilled water to remove the reaction solution, and further washed by placing the sheet in a sealed container containing a cleaning solution (1 M saline to which 0.05% Tween 20 has been added) and violently agitating the cleaning solution. After thoroughly draining the sheet, 10 microliters of substrate solution (1 mg/ml 5-bromo-4-chloro-3-indoryl phosphate BCIP), 1 mM magnesium chloride, 0.1 M boric acid-sodium carbonate buffer solution, pH 9.8) were added dropwise and left to stand at room temperature for 30 minutes until the solution was dried. The CEA concentration of the specimens was quantitated by measuring the reflectivity within the circular frame with a reflectivity meter (Color Measuring System SZ-80, manufactured by Nihon Denshoku Industries K.K.) using contrasts prepared by coating CEA standard solutions (CEA concentration: 0, 50, 100, 200, and 500 ng/ml) instead of the specimen.

(c-2) Procedure 2

10 microliter portions of ALP-labelled antibody No. 1 were applied dropwise within the circular frames of the antibody-insolubilized sheet prepared in (a), 5 microliter portions of the specimens used in (c-1) were coated thereon, and allowed to react for 30 minutes at room temperature. The sheet was washed with distilled water to remove the reaction solution, and further washed with the cleaning solution. The CEA concentration of the specimens was quantitated by repeating the procedure of (c-1).

(c-3) Procedure 3

5 microliters of each of the specimens used in (c-1) were mixed with 10 microliters of the ALP-labelled antibody No. 1. The mixtures were coated within the circular frame of the antibody-insolubilized sheet prepared in (a), and allowed to react for 30 minutes at room temperature. The sheet was washed with distilled water to remove the reaction solution, and further washed with the cleaning solution. The CEA concentration of the specimens was quantitated by repeating the procedure of (c-1).

The results obtained in the above-described three procedures are shown in Table 5. The results obtained were consistent among these three procedures.

Table 5

| Specimen No. | CEA concentration, ng/ml | | |
|---|---|---|---|
| | Procedure 1 | Procedure 2 | Procedure 3 |
| 1 | 0 | 100 | 200 |
| 2 | 0 | 100 | 200 |
| 3 | 0 | 100 | 200 |

Example 4: Assay of CEA by a procedure wherein the nipple discharge is directly adsorbed onto the polypropylene sheet

(a) Preparation of the enzyme-labelled antibody

An HRPO-labelled anti-CEA antibody (hereinafter referred to as HRPO-labelled antibody No. 10) was prepared by repeating the procedure of Example 1, (b) using antibody No. 10.

(b) Measurement of CEA

From patients suffering from abnormal nipple discharge, 18 specimens of the nipple discharge were collected and used for measuring CEA.

5 microliters of each of the specimens were applied onto the polypropylene sheets. The sheets were then incubated at 37°C for 1 hour, immersed in PBS to which 0.5% powdered skim milk has been added, and further incubated overnight at 4°C. Moisture was thoroughly absorbed off the sheets by using filter papers. Said polypropylene was immersed in a solution of HRPO-labelled antibody No. 10 diluted by a

13

factor of a thousand with 1% BSA-added PBS, and incubated at room temperature for 1 hour. After absorbing moisture off the sheets by using filter papers, the sheets were placed in a sealed container containing the cleaning solution (1 M saline to which 0.05% Tween 20 has been added), and washed for 1 hour by agitating the solution. The cleaning solution was changed in every 15 minutes. The thus washed polypropylene sheets were thoroughly drained, immersed in the substrate solution (0.5 mM TMB, 0.01% $H_2O_2$), and incubated at room temperature for 15 minutes. The specimens wherein the color had developed were determined to be +, and those wherein no color developed were determined to be -. The thus obtained results are shown in Table 6 together with the measurements of the same specimens obtained by Elmotech-CEA. A good correlation was observed between the measurements of both methods.

Table 6

| Specimen | Present method (qualitative) | Elmotech-CEA (quantitative) |
|---|---|---|
| A | + | 285 ng/ml |
| B | + | 650 |
| C | + | 445 |
| D | + | 343 |
| E | + | 605 |
| F | + | 555 |
| G | - | 96 |
| H | - | 36 |
| I | - | 18 |
| J | - | 31 |
| K | - | 8 |
| L | - | 10 |
| M | - | 96 |
| N | - | 46 |
| O | - | 70 |
| P | - | 5 |
| Q | - | 11 |
| R | - | 0 |

As apparent from the above-described Examples, the method and the device for assaying the target substance in a minute amount of the nipple discharge according to the present invention may be used for conveniently assaying a quite minute amount of specimen allowing for a qualitative or semi-quantitative reaction without requiring any particular measuring apparatus. Therefore, the method and the device of the present invention may readily be used for group examination, mass-screening, and the like. Further, the method and the device of the present invention enables a detection of the breast cancer at an early stage.

## Claims

1. Method for assaying a target substance in a minute amount of nipple discharge wherein concentration of the target substance in the nipple discharge is assayed by means of an antigen-antibody reaction using an antibody or a fragment thereof against said target substance, and said antigen-antibody reaction is effected on a sheet-form solid phase.

2. The method according to claim 1 wherein the antibody or the fragment thereof against the target substance is insolubilized on said sheet-form solid phase.

3. The method according to claim 1 or 2 wherein said concentration of the target substance in the nipple discharge is assayed by

(a) insolubilizing and immobilizing the antibody and/or the fragment thereof against the target substance onto said sheet-form solid phase,

(b) collecting a specimen of the nipple discharge, and applying said specimen onto a predetermined reaction area of said solid phase to contact the specimen with the antibody and/or the fragment thereof insolubilized within said area,

(c) applying a labelled substance onto the predetermined reaction area before or after the drying and solidification of said specimen on said solid phase so that the labelled substance becomes in contact with said specimen, said labelled substance comprising said antibody and/or the fragment thereof against the target substance having an identifiable labelling agent bound thereto,

(d) rinsing said solid phase to remove portion of the specimen and the labelling substance which failed to bind with the antibody and/or the fragment thereof insolubilized onto the solid phase, and

(e) assaying the concentration of said target substance in the nipple discharge from the amount of the labelling substance bound to said solid phase.

4. The method according to claim 1 or 2 wherein said contactings of the specimen to the antibody against the target substance and/or the fragment thereof insolubilized onto said sheet-form solid phase, and the labelled substance to the specimen are simultaneously effected.

5. The method according to claim 1 or 2 wherein the nipple discharge specimen is mixed with the labelled substance comprising the antibody and/or the fragment thereof against the target substance in the nipple discharge having the identifiable labelling agent bound thereto, and the resulting mixture is contacted with said target substance insolubilized on said sheet-form solid phase.

6. The method according to claim 3 wherein said target substance in the nipple discharge is a tumor-associated antigen.

7. The method according to claim 3 wherein the antibody and/or the fragment thereof against the target substance is an antibody and/or a fragment thereof against the tumor-associated antigen.

8. The method according to claims 6 or 7 wherein said tumor-associated antigen is CEA (carcinoembryonic antigen).

9. The method according to claim 3 wherein said sheet-form solid phase is a substantially liquid-impermeable plastic sheet.

10. The method according to claim 9 wherein said plastic is selected from the group consisting of polyester, polypropylene, and polyethylene.

11. The method according to claim 3 wherein the sheet-form solid phase onto which the antibody and/or the fragment thereof is insolubilized is coated with a substance which does not participate in the antigen-antibody reaction between the target substance and the antibody and/or the fragment thereof against said target substance.

12. The method according to claim 3 wherein said nipple discharge specimen is collected by directly bringing said sheet-form solid phase into contact with the nipple.

13. The method according to claim 3 wherein said nipple discharge specimen is collected by means of a collecting device.

14. The method according to claim 3 wherein said identifiable labelling agent is an enzyme, and said measurement of the labelled substance is carried out by assaying an amount of a substance which increases or decreases as a result of an enzyme reaction of said enzyme.

15. The method according to claim 3 wherein the concentration of said target substance is semiquantitatively or qualitatively determined by the naked eye.

16. The method according to claim 3 wherein the concentration of said target substance is quantitatively determined by physical means.

17. A device for assaying a target substance in a minute amount of nipple discharge comprising a sheet-form solid phase having at least one reaction area defined thereon for an antigen-antibody reaction between an antibody and the target substance in the nipple discharge.

18. The device according to claim 17 wherein an antibody and/or a fragment thereof against said target substance is insolubilized onto said reaction area.

19. The device according to claim 18 wherein said sheet-form solid phase has a labelled substance comprising the antibody and/or the fragment thereof against said target substance and an identifiable labelling agent bound thereto.

20. The device according to claim 17 or 18 wherein said target substance in the nipple discharge is a tumor-associated antigen.

21. The device according to claim 17 or 18 wherein the antibody and/or the fragment thereof against the target substance is an antibody and/or a fragment thereof against the tumor-associated antigen.

22. The device according to claims 20 or 21 wherein said tumor-associated antigen is CEA (carcinoembryonic antigen).

23. The device according to claim 17 or 18 wherein said sheet-form solid phase is a substantially liquid-impermeable plastic sheet.

15

24. The device according to claim 23 wherein said plastic is selected from the group consisting of polyester, polypropylene, and polyethylene.

25. The device according to claim 17 or 18 wherein said reaction area on said sheet-form solid phase is surrounded by a ridge or a frame provided on said sheet-form solid phase.

26. The device according to claim 17 or 18 wherein said reaction area on said sheet-form solid phase is a recess provided in said sheet-form solid phase.

27. The device according to claim 19 wherein said identifiable labelling agent is selected from the group consisting of a pigment, radio-isotope, enzyme, and fluorescent material.

28. The device according to claim 18 wherein said reaction area is coated with a substance which does not participate in the antigen-antibody reaction between the target substance and the antibody and/or the fragment thereof against said target substance.

## F I G . 1

## F I G . 2a

## F I G . 2b

F I G . 3a

F I G . 3b

F I G . 4a

F I G . 4b

**F I G . 5a**

**F I G . 5b**